# EUROPÄISCHE PATENTSCHRIFT

(11) **EP 2 943 250 B1**
(45) Veröffentlichungstag und Bekanntmachung des Hinweises auf die Patenterteilung: **27.09.2017**
(21) Anmeldenummer: 13811835.1
(22) Anmeldetag: 10.12.2013
(51) Int. Cl.: A61N 1/375, A61N 1/05, A61N 1/08, A61N 1/36

(54) **VORRICHTUNG ZUR KONTAKTIERUNG UND/ODER ELEKTROSTIMULATION VON BIOLOGISCHEM GEWEBE**
DEVICE FOR THE CONTACTING AND/OR ELECTROSTIMULATION OF BIOLOGICAL TISSUE
DISPOSITIF DESTINÉ À LA MISE EN CONTACT ÉLECTRIQUE ET/OU À L'ÉLECTROSTIMULATION D'UN TISSU BIOLOGIQUE

(30) Priorität: 11.01.2013 DE 102013100256
(43) Veröffentlichungstag der Anmeldung: 18.11.2015
(73) Patentinhaber: Retina Implant AG, 72770 Reutlingen (DE)
(72) Erfinder: KOKELMANN, Martin, 72658 Bempflingen (DE)
(74) Vertreter: Witte, Weller & Partner Patentanwälte mbB
(86) Internationale Anmeldenummer: PCT/EP2013/076096
(87) Internationale Veröffentlichungsnummer: WO 2014/108266

(56) Entgegenhaltungen:
- EP-A2- 2 066 402
- DE-A1-102006 021 258
- US-A1- 2006 069 416
- US-A1- 2006 173 511
- US-A1- 2007 142 877
- US-A1- 2007 207 569
- US-A1- 2010 106 227

## Beschreibung

Die vorliegende Erfindung betrifft eine Vorrichtung zur Kontaktierung und/oder Elektrostimulation von biologischem Gewebe, mittels zumindest einer Elektrode, wobei die Vorrichtung zumindest eine erste Einheit, an der die zumindest eine Elektrode vorgesehen ist, und die zur Implantation in einen menschlichen oder tierischen Körper ausgelegt ist, eine zweite Einheit, die eine Spannungsversorgung zur Versorgung der ersten Einheit mit elektrischer Energie bereitstellt, sowie zumindest eine erste und eine zweite Leiterbahn zur Verbindung der Spannungsversorgung mit der ersten Einheit aufweist, wobei die Leiterbahnen jeweils elektrisch mit der ersten und der zweiten Einheit verbunden sind und auf unterschiedlichen Spannungspotentialen liegen.

Derartige Vorrichtungen sind aus dem Stand der Technik umfangreich bekannt.

Die als Implantat ausgebildete erste Einheit dient beispielsweise als Retinaimplantat zur Stimulation von Nervenzellen im Auge, als Cochlea-Implantat zur Stimulation des menschlichen Innenohrs, als Neuroimplantat zur Stimulation oder Ableitung elektrischer Signale aus Regionen des Gehirns, oder ganz allgemein zur Stimulation oder Ableitung von elektrischen Signalen von biologischem Gewebe.

Die Implantate müssen dabei jeweils mit elektrischer Energie versorgt werden, wozu sie mit einer eine zweite Einheit bildenden Versorgungseinheit verbunden sind, die ebenfalls implantiert oder außerhalb des Körpers angeordnet wird. Zwischen den beiden Einheiten sind stromführende Leiterbahnen aus edlen Metallen wie Gold, Platin oder Titan vorgesehen, die durch eine Hülle aus Kunststoff vor Körperflüssigkeiten geschützt werden.

Bei einigen Implantaten wird die Verbindung zwischen der ersten und der zweiten Einheit über Drähte realisiert, in denen elektrische Signale transportiert und die elektrischen Potentiale der Versorgungsspannung zur Verfügung gestellt werden. Die Drähte sind mit einem Isolator aus Kunststoff überzogen, um Kurzschlüsse zu vermeiden. Hierzu wird meist Silikon verwendet, wodurch die Drähte nicht nur gegeneinander isoliert sondern gleichzeitig auch mit einem guten mechanischen Schutz versehen werden. Das die Drähte umgebende Silikon hat eine ausreichende Dicke, um auch gegen die elektronikfeindliche feuchte Umgebung hinreichenden Schutz zu bieten.

Wenn dagegen Retinaimplantate oder Neuroimplantate mit elektrischer Energie versorgt werden sollen, so sind die räumlichen Gegebenheiten sehr viel enger, weil beispielsweise im Auge nur wenig Freiraum zur Verfügung steht, um vergleichbar dicke Kabel zu verlegen.

Aus diesem Grund werden zur Versorgung von Retinaimplantaten flexible Verbindungsbänder verwendet, die nach Art einer schmalen, länglichen, flexiblen Leiterplatte ausgebildet sind. Die Leiterbahnen werden dabei zwischen zwei biokompatible Kunststoffschichten eingebettet. Als Materialien für die Kunststoffschichten kommen Polyimide, Parylene, LCP (liquid crystal polymer) oder Silikone infrage.

Derartige Retinaimplantate sind beispielsweise aus der WO 2004/ 067088 A1, der WO 2008/037363 A2 sowie der DE 10 2006 021 258 A1 bekannt, auf deren Inhalt hiermit ausdrücklich verwiesen wird.

Die bekannten Retinaimplantate werden in den subretinalen oder epiretinalen Raum des Auges implantiert, wobei die zweite Einheit auf demselben flexiblen Substrat ausgebildet sein kann wie die erste Einheit, wie es beispielsweise in der DE 10 2006 021 258 A1 beschrieben ist.

Die zweite Einheit kann dabei einen Infrarot-Empfänger enthalten, der einfallendes IR-Licht in elektrische Energie zur Versorgung der ersten Einheit umwandelt.

Es ist auch bekannt, die erste und die zweite Einheit auf zwei unterschiedlichen Substraten auszubilden und diese beiden Substrate über ein flexibles Verbindungsband miteinander zu verbinden und beide dann ins Auge zu implantieren.

In anderen Anwendungen ist die zweite Einheit eine externe Einheit, die außerhalb des Körpers, beispielsweise an der Stirn, geeignet befestigt wird, wie dies beispielsweise aus der WO 2008/037363 A2 oder der DE 10 2006 021 258 A1 bekannt ist.

Die externe zweite Einheit sowie die implantierte erste Einheit sind dann ebenfalls über ein flexibles Verbindungsband miteinander verbunden, das beispielsweise ebenfalls im subretinalen Raum verläuft.

Die als Versorgungseinheit ausgebildete externe zweite Einheit kann dabei die elektrische Energie für die als Implantat ausgebildete erste Einheit aus Batterien beziehen, über externe Kabel empfangen oder aus elektromagnetischer Energie gewinnen, wie dies beispielsweise in der WO 00/67676 A1 bekannt ist.

Den insoweit beschriebenen aktiven Retinaimplantaten ist gemeinsam, dass sie eine Vielzahl von Stimulationselektroden aufweisen, die elektrische Stimulationssignale an zu kontaktierende Zellen der Retina abgeben. Ferner ist eine Vielzahl von Bildelementen vorgesehen, die einfallendes Licht in die Stimulationssignale umwandeln. Wegen weiterer Details wird auf die insoweit zitierten Druckschriften verwiesen.

Die beschriebenen Retinaimplantate haben sich aus medizinischer Sicht bereits bewährt, ihre Verweildauer im Auge ist jedoch aus technischer Hinsicht noch begrenzt, weil es nämlich nach einer Verweildauer von wenigen 100 Stunden zwischen den beiden Leiterbahnen, die die erste und die zweite Einheit zum Zwecke der Spannungsversorgung miteinander verbinden, zu Kurzschlüssen oder Leitungsunterbrechungen kommt, so dass die Spannungsversorgung des Implantates nicht mehr sichergestellt ist und es explantiert bzw. ausgetauscht werden muss.

Die US 2010/0106227 A1 beschreibt einen Implantierbaren Herzschrittmacher, dessen Elektrode über eine Koaxialleiteranordnung mit dem eigentlichen implantierten Gerät verbunden ist. Die Koaxialleiteranordnung weist einen inneren Leiter auf, der konzentrisch in einem äußeren Leiter verläuft. Innen in dem äußeren Leiter ist neben dem inneren Leiter eine Steuerleitung geführt. Die Steuerleitung und der innere Leiter sind durch in dem äußeren Leiter angeordnetes Isolationsmaterial voneinander getrennt. Der äußere Leiter und der innere Leiter transportieren Anregungsimpulse von einem Impulsgenerator des Gehäuses zu der Elektrode an der Spitze der Koaxialleiteranordnung. An der Spitze der Koaxialleiteranordnung ist ferner ein Schalter vorgesehen, der mit der Steuerleitung verbunden ist, und über den die Elektrode zu- oder abgeschaltet werden kann.

Vor diesem Hintergrund liegt der vorliegenden Erfindung die Aufgabe zugrunde, eine Vorrichtung der eingangs genannten Art auf konstruktiv einfache Weise als Retinaimplantat einzusetzen und so weiterzubilden, dass die maximale Verweildauer im implantierten Zustand erhöht wird.

Erfindungsgemäß wird diese Aufgabe bei der eingangs erwähnten Vorrichtung dadurch gelöst, dass die erste Einheit eine Stimulationseinheit für die Retina ist, die eine Vielzahl von Bildelementen umfasst, die einfallendes Licht in elektrische Signale umwandeln, die über Stimulationselektroden (22) abgegeben werden, und dass zumindest eine funktional nicht in die Versorgung der ersten Einheit mit elektrischer Energie eingebundene Zusatzleiterbahn vorgesehen ist, die räumlich zwischen der ersten und der zweiten Leiterbahn angeordnet ist.

Die der Erfindung zugrunde liegende Aufgabe wird auf diese Weise vollkommen gelöst.

Unter einer "funktional nicht in die Versorgung mit elektrischer Energie eingebundenen Zusatzleiterbahn" wird im Rahmen der vorliegenden Erfindung eine Leiterbahn verstanden, die technisch so ausgebildet sein kann, wie die funktional wirksamen Leiterbahnen, aber weder unmittelbar mit der Masse noch unmittelbar mit einem Spannungspotential einer Versorgungsspannung verbunden ist. Für die Zwecke der Spannungsversorgung ist die Zusatzleiterbahn also ohne elektrische Funktion, sie verlängert jedoch auf nicht zu erwartende Weise die Verweildauer der neuen Vorrichtung im implantierten Zustand.

Die Erfinder haben nämlich nach dem elektrischen Versagen wieder explantierte Implantate untersucht, um den Fehler des Kurzschlusses bzw. der Leitungsunterbrechung zu analysieren. Dabei hat sich herausgestellt, dass die umgebende Körperflüssigkeit die Kunststoffschichten zum Quellen bringt, wodurch Poren entstehen, in die Flüssigkeit und in der Flüssigkeit gelöste Ionen eindringen können, die auch bis zu den Leiterbahnen gelangen.

Unter elektrischer Spannung kommt es dann zu einem elektrolytischen Prozess, der zu einer Zersetzung des Leiterbahnmaterials führt. Der Einsatz von edlen Metallen kann diesen Prozess nur bedingt stoppen.

Auf dem flexiblen Substrat des Verbindungsbandes sind die Leiterbahnen meist parallel angeordnet, so dass es zwischen ihnen zu einer induktiven und kapazitiven Kopplung kommt, was einen geringen Leckstrom zwischen den Leiterbahnen verursacht. Dieser Effekt ist als Elektromigration bekannt, er führt zu einem Materialabtrag an einer der beiden Leiterbahnen.

Die identifizierten Probleme, die zu dem erkannten Kurzschluss oder der Leitungsunterbrechung zwischen den Leiterbahnen führen, beruhen also zum einen auf dem Quellen der Isolationsschichten, zwischen denen die Leiterbahnen angeordnet sind, und zum anderen auf der Elektromigration, die zum Materialabtrag führt.

Die Erfinder der vorliegenden Anmeldung sind nun nicht den Weg gegangen, durch mechanische Verstärkung der Substrate oder der Verbindung zwischen dem oberen und dem unteren Substrat und/oder durch breitere Abmaße der Leiterbahnen sowie durch größere Abstände zwischen den parallel verlaufenden Leiterbahnen diesen Effekt zu verringern, sondern haben sozusagen eine dritte Leiterbahn, nämlich die Zusatzleiterbahn, zwischen den beiden vorhandenen Leiterbahnen angeordnet.

Der Effekt der Zusatzleiterbahn zwischen der ersten und der zweiten Leiterbahn beruht dabei nicht vorrangig darauf, dass durch das Einfügen der Zusatzleiterbahn der Abstand zwischen der ersten und der zweiten Leiterbahn vergrößert wird, denn der in Versuchen der Erfinder gemessene Effekt ist deutlich größer, als es durch die Vergrößerung des Abstandes erzielbar war.

Zudem hat sich gezeigt, dass die Zusatzleiterbahn eine geringere Breite als die Leiterbahnen aufweisen kann, sie beträgt beispielsweise nur 10% bis 20% der Breite der Leiterbahnen, weist aber die gleiche Dicke auf.

Dies hat den Vorteil, dass die Breite des Verbindungsbandes durch die Einfügung der Zusatzleiterbahn nicht zu sehr zunimmt, so dass die Implantation in das Auge nicht beeinträchtigt wird.

Die erfindungsgemäße Verwendung von Zusatzleiterbahnen ist insbesondere bei Retinaimplantaten von Vorteil, weil bei diesen sehr beengte räumliche Vorgaben zu berücksichtigen sind, so dass mechanische Schutzmaßnahmen nur problematisch zu realisieren sind.

In einem bevorzugten Ausführungsbeispiel weisen die Leiterbahnen und Zusatzleiterbahnen eine Dicke auf, die zwischen 0,1 um und 20 um liegt, wobei die Leiterbahnen eine Breite aufweisen, die zwischen 10 um und 1 mm liegt.

In ersten Versuchen konnten die Erfinder zeigen, dass bei Implantaten bzw. Verbindungsbändern ohne Zusatzleiterbahn ein Kurzschluss nach einigen 100 bis maximal ca. 1.000 Stunden in einer flüssigen Umgebung zu beobachten war, der die physiologischen Bedingungen des implantierten Zustandes nachahmt. Wird dagegen die Zusatzleiterbahn verwendet, so wurde in einem Fall die Verweildauer auf über 2.500 Stunden erhöht, während bei einem zweiten Versuch die Messung nach 4.000 Stunden abgebrochen wurde, da sich keine negativen Effekte zeigten.

Bisher liegt den Erfindern keine verifizierte Erklärung für diesen Effekt vor, es wurde jedoch beobachtet, dass das Quellen der Isolationsschichten zwar nicht beseitigt wurde, der Kontakt zwischen den Isolationsschichten aber über längere Zeit stabil blieb. Ferner scheint durch die Zusatzleiterbahn die Ausbildung von elektromagnetischen Feldern zwischen der ersten Leiterbahn und der zweiten Leiterbahn begrenzt oder verändert zu werden, zumindest war zu beobachten, dass die Effekte der Elektromigration bei den Versuchen mit Zusatzleiterbahn deutlich verringert waren.

Wider Erwarten führt also eine elektrisch inaktive Zusatzleiterbahn, die zwischen den beiden Leiterbahnen angeordnet ist, dazu, dass die Verweildauer der neuen Vorrichtung in physiologischer Umgebung deutlich verbessert wird, ohne dass die seitlichen Abmaße der Leiterbahnen sowie des Zwischenraumes zwischen den beiden Leiterbahnen stark vergrößert werden müssen, was bei der Anwendung für Retinaimplantate oder Neuroimplantate sowieso nur begrenzt möglich wäre.

Die vorliegende Erfindung kann auch bei Vorrichtungen eingesetzt werden, bei denen zwischen der ersten und der zweiten Einheit mehr als zwei Leiterbahnen verlaufen, wobei zwischen je zwei benachbarten Leiterbahnen zumindest eine Zusatzleiterbahn vorgesehen ist. Dies kann beispielsweise dann erforderlich sein, wenn Versorgungsspannungen verschiedener Polarität und/oder Spannungspegel übertragen werden sollen.

Unter Leiterbahn und Zusatzleiterbahnen werden im Rahmen der vorliegenden Erfindung zwischen zwei in der Regel flexiblen Substraten, in der Regel Kunststoffschichten, eingebettete Leiterbahnen verstanden, wie sie in elektronischen Schaltungen zur Anbindung der Spannungsversorgung verwendet werden. Diese Leiterbahnen werden auf die Substrate aufgedruckt oder anderweitig auf den Substraten strukturiert, beispielsweise durch Sputtern, PVD (physical vapour deposition) oder galvanisch. Die so hergestellten Leiterbahnen haben in der Regel eine Breite von weniger als 1 mm und eine Dicke von weniger als 100 um (Mikrometer).

Dabei ist es bevorzugt, wenn die Zusatzleiterbahn potentialfrei geschaltet ist.

Hier ist von Vorteil, dass die Zusatzleiterbahn an ihren beiden Enden nicht angeschlossen werden muss, also als blinde Leiterbahn verläuft, die beliebig floaten kann.

Bei dieser Maßnahme ist von Vorteil, dass sie konstruktiv sehr einfach zu realisieren ist, zwischen den beiden Leiterbahnen muss lediglich eine Zusatzleiterbahn angeordnet sein, die jedoch nicht weiter verschaltet werden muss.

Alternativ kann die Zusatzleiterbahn gegenüber der ersten und/oder der zweiten Leiterbahn auch einen elektrischen Widerstand aufweisen, der größer als 100 kOhm ist.

Diese Maßnahme ist dann von Vorteil, wenn floatende Zusatzleiterbahnen in der Vorrichtung stören.

Dabei ist es bevorzugt, wenn die Zusatzleiterbahn über zumindest 50 %, vorzugsweise über zumindest 90 %, weiter vorzugsweise über zumindest der gesamten Länge der ersten und zweiten Leiterbahn zwischen diesen verläuft.

Diese Maßnahme ist konstruktiv von Vorteil, denn in Abhängigkeit von den jeweiligen geometrischen Gegebenheiten können die Zusatzleiterbahnen nur über einen Teil oder über der gesamten Länge zwischen der ersten und der zweiten Leiterbahn verlaufen.

Weiter ist es bevorzugt, wenn neben der ersten Leiterbahn eine zweite Zusatzleiterbahn derart vorgesehen ist, dass die erste Leiterbahn zwischen der ersten und der zweiten Zusatzleiterbahn verläuft, wobei vorzugsweise neben der zweiten Leiterbahn eine dritte Zusatzleiterbahn derart vorgesehen ist, dass die zweite Leiterbahn zwischen der ersten und der dritten Zusatzleiterbahn verläuft, wobei weiter vorzugsweise zumindest zwei Zusatzleiterbahnen mit ihren äußeren Enden miteinander verbunden sind.

Weiter vorzugsweise ist zwischen der ersten und der zweiten Leiterbahn eine weitere Zusatzleiterbahn vorgesehen, wobei vorzugsweise die erste und die zweite Zusatzleiterbahn sowie die weitere und die dritte Zusatzleiterbahn an ihren äußeren Enden miteinander verbunden sind.

Die Erfinder der vorliegenden Anmeldung haben erkannt, dass sich durch diese konstruktiv einfach zu realisierenden Maßnahmen die Schutzfunktion weiter erhöht.

Die Verbindung der Zusatzleiterbahnen miteinander kann dabei über Kontaktbrücken erfolgen, die über die Leiterbahnen geführt sind, gegenüber denen sie elektrisch isoliert sind. Umgekehrt ist es auch möglich, die Leiterbahnen über Brücken weiterzuführen oder innen in dem geschlossenen Ring oder Rechteck aus Zusatzleiterbahnen in Anschlussflächen enden zu lassen, von denen gebondete Drahtverbindungen zu der ersten oder zweiten Einheit führen.

Weiter ist es bevorzugt, wenn die Vorrichtung ein flexibles Verbindungsband umfasst, das mechanisch mit der ersten und der zweiten Einheit verbunden ist und auf dem die Leiterbahnen sowie die Zusatzleiterbahn oder die Zusatzleiterbahnen angeordnet sind.

Die zweite Einheit kann dann entweder räumlich beabstandet im Auge oder Gehirn oder auch außerhalb des Körpers liegen, wie dies aus den eingangs erwähnten Druckschriften bekannt ist. Wenn die erste Einheit und die zweite Einheit dagegen auf einem gemeinsamen flexiblen Substrat ausgebildet sind, verlaufen die Leiterbahnen auf diesem einheitlichen Substrat, wobei der erfindungsgemäße Schutzeffekt dann zum Tragen kommt, wenn die Leiterbahnen auf einem Abschnitt des gemeinsamen Substrates ausgebildet sind, der wie ein erfindungsgemäßes Verbindungsband wirkt und die Zusatzleiterbahnen aufweist, da ohne die erfindungsgemäß vorgesehenen Zusatzleiterbahnen nur eine begrenzte Lebensdauer der Vorrichtungen im implantierten Zustand zur Verfügung steht.

In der Regel verlaufen die Leiterbahnen und die Zusatzleiterbahn oder die Zusatzleiterbahnen parallel zueinander, was den Vorteil mit sich bringt, dass das Verbindungsband sehr schmal ausgebildet sein kann, so dass es für den Einsatz bei Retinaimplantaten den Vorteil aufweist, wenig Platz im Auge, insbesondere im subretinalen Raum, zu beanspruchen.

Dabei ist es bevorzugt, wenn die erste Leiterbahn mit einer Masse und die zweite Leiterbahn mit einer Gleichspannung der Versorgungsspannung verbunden sind. Die Erfindung ist jedoch auch anwendbar bei anderen Versorgungsleitungen mit hohen Potentialunterschieden, beispielsweise bei einer Versorgungsspannung, bei der die erste Leiterbahn auf -5 Volt und die zweite Leiterbahn auf + 5 Volt liegt.

Insbesondere dann, wenn die beiden parallel zueinander verlaufenden Leiterbahnen mit Masse- bzw. Spannungspotential belegt sind, kommt es nach Erkenntnis der Erfinder zu einem schnellen Einsetzen der Elektromigration, insbesondere dann, wenn die Leiterbahnen über längere Strecken parallel zueinander verlaufen.

Bei dieser kritischen Konstruktion wirkt sich der erfindungsgemäße Effekt der Zusatzleiterbahn besonders vorteilhaft aus.

Allgemein ist es bevorzugt, wenn das Verbindungsband eine untere, elektrisch isolierende Materialschicht, auf der die Leiterbahnen und die Zusatzleiterbahn oder die Zusatzleiterbahnen aufgebracht sind, und eine über den Leiterbahnen und der Zusatzleiterbahn oder den Zusatzleiterbahnen vorgesehene obere, elektrisch isolierende Materialschicht aufweist, wobei die isolierenden Materialschichten vorzugsweise aus Polyimid gefertigt sind.

Hier ist von Vorteil, dass mit Polyimid ein besonders biokompatibles Material als Isolationsschicht verwendet wird, wobei die isolierenden Materialschichten auch die hinreichende Flexibilität aufweisen, um das Verbindungsband im Auge oder aus dem Auge heraus verlegen zu können.

Die zweite Einheit ist dabei entweder eine implantierbare Versorgungseinheit, die einfallende Strahlungsenergie in elektrische Energie zur Versorgung der Stimulationseinheit für die Retina umwandelt, wie es aus der eingangs erwähnten WO 2004/067088 A1 bekannt ist, oder eine externe Versorgungseinheit, die elektrische Energie zur Versorgung des Stimulationseinheit für die Retina bereitstellt, wie es beispielsweise aus der eingangs erwähnten DE 10 2006 021 258 A1 oder der WO 2000/67676 A1 bekannt ist.

Weitere Vorteile ergeben sich aus der Beschreibung und der beigefügten Zeichnung.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: eine schematische Darstellung einer vollständig implantierbaren Vorrichtung zur Elektrostimulation der Retina, in nicht maßstabsgetreuer Darstellung;
- Fig. 2: eine schematische Darstellung eines menschlichen Auges, in das die Vorrichtung gemäß Fig. 1 eingesetzt ist, ebenfalls nicht maßstabsgetreu;
- Fig. 3: eine weitere, nicht maßstabsgetreu dargestellte Vorrichtung zur Elektrostimulation der Retina, mit einer implantierbaren Stimulationseinheit und einer externen Versorgungseinheit;

- Fig. 4: eine schematische Darstellung eines menschlichen Auges wie in Fig. 1, jedoch mit der Vorrichtung gemäß Fig. 3;
- Fig. 5: eine schematische Draufsicht auf eine Vorrichtung mit einer implantierbaren ersten Einheit und einer zweiten Einheit, die über ein Verbindungsband miteinander verbunden sind;
- Fig. 6: eine Schnittdarstellung der Vorrichtung aus Fig. 5, gesehen längs der Linie VI-VI aus Fig.5;
- Fig. 7: ein erstes Ausführungsbeispiel eines Verbindungsbandes, wie es für eine Vorrichtung aus den Fig. 1, 3 und 5 verwendbar ist, in schematischer Draufsicht;
- Fig. 8: ein zweites Ausführungsbeispiel eines Verbindungsbandes, in einer Darstellung wie in Fig. 7; und
- Fig. 9: ein drittes Ausführungsbeispiel eines Verbindungsbandes, in einer Darstellung wie in Fig. 7;

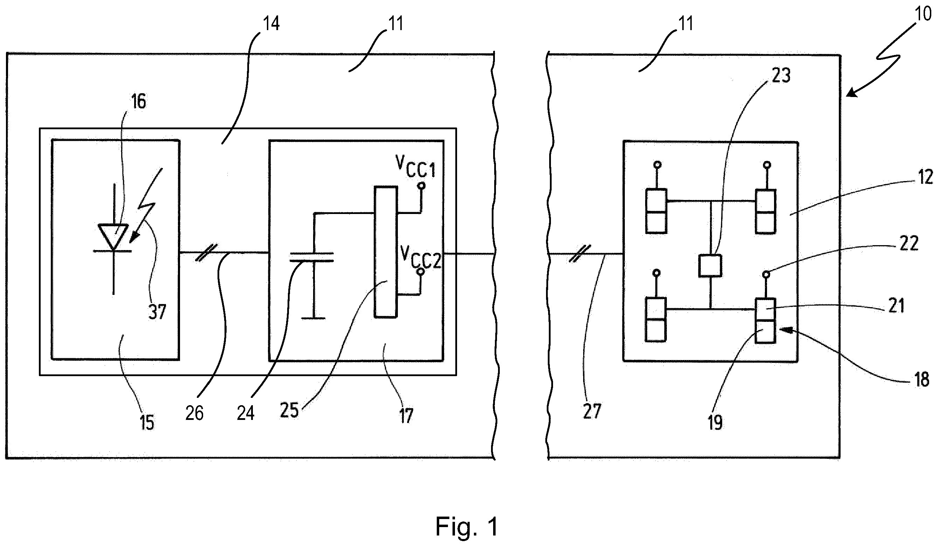

In Fig. 1 ist schematisch eine Vorrichtung 10 zur Elektrostimulation der Retina dargestellt, wobei die Abmaße nicht maßstabgetreu wiedergegeben sind.

Die Vorrichtung 10 ist auf einem Substrat in Form einer flexiblen Folie 11 ausgebildet, auf der eine Stimulationseinheit 12 sowie eine Versorgungseinheit 14 angeordnet sind. Die Versorgungseinheit 14 umfasst einen IR-Empfänger 15, der ein oder mehrere fotovoltaische Elemente 16 enthält, die auftreffendes IR-Licht in elektrische Spannung umwandeln. Die so eingekoppelte Fremdenergie wird an eine Spannungserzeugung 17 übergeben, die eine Versorgungsspannung für die Stimulationseinheit 12 erzeugt.

Die Stimulationseinheit 12 umfasst beispielsweise in Reihen und Spalten angeordnete Bildelemente 18, von denen in Fig. 1 der Übersichtlichkeit halber lediglich vier dargestellt sind. Jedes Bildelement 18 umfasst eine logarithmische Bildzelle 19 für lokale Bildhelligkeit sowie einen Verstärker 21, der an seinem Ausgang mit einer Stimulationselektrode 22 verbunden ist. Ferner ist auf der Stimulationseinheit 12 eine Bildzelle 23 für globale Helligkeit vorgesehen, die mit den Verstärkern 21 sämtlicher Bildelemente 18 auf der Stimulationseinheit 12 verbunden ist. Es versteht sich, dass die Stimulationseinheit 12 mehrere globale Bildzellen 23, oder aber auch nur eine einzige davon, umfassen kann.

Die Spannungserzeugung 17 weist ein Speicherelement 24 auf, in dem die von dem IR-Empfänger 15 aufgenommene Fremdenergie gespeichert wird. Das Speicherelement 24 ist mit einem Schaltungsteil 25 verbunden, das aus den über eine Mehrfachleitung 26 empfangenen Ausgangssignalen des IR-Empfängers beispielsweise zwei verschiedene Versorgungsspannungen V_{cc1} und V_{cc2} erzeugt. Über mehre Leiterbahnen 27 ist die Versorgungseinheit 14 mit der Sensoreinheit 12 verbunden. Wegen weiterer Details dieser Vorrichtung wird auf die eingangs erwähnte DE 10 2006 021 258 A1 verwiesen.

Aus Fig. 1 ist zu entnehmen, dass Stimulationseinheit 12 und Versorgungseinheit 14 auf der gemeinsamen Folie 11 als räumlich getrennte Einheiten angeordnet sind, die über die Leiterbahnen 27 miteinander verbunden sind. Alternativ können Stimulationseinheit 12 und Versorgungseinheit 14 auch mechanisch getrennt auf eigenen Substraten ausgebildet sein und über ein Verbindungsband mechanisch miteinander verbunden sein, auf dem die Leiterbahnen 27 zur Versorgung der Stimulationseinheit 12 mit elektrischer Energie verlaufen.

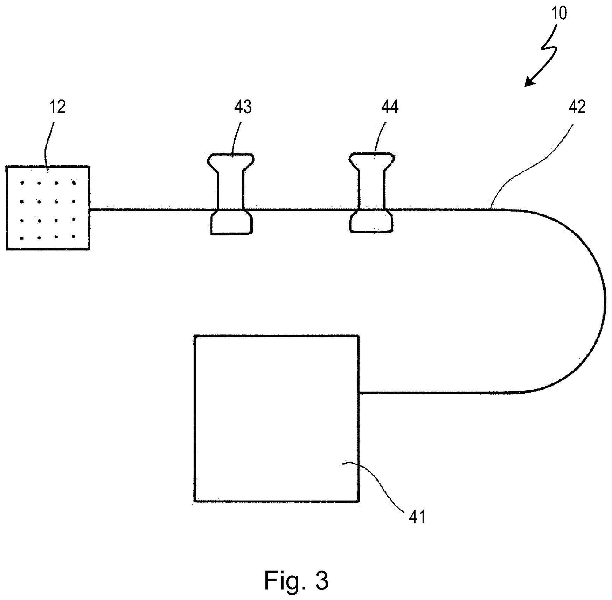

Die Versorgungseinheit 14 kann dabei ebenfalls ins Auge implantiert werden, wie es bei der Vorrichtung 10 aus Fig. 1 vorgesehen ist, oder außerhalb des Auges angeordnet werden, wie es bei der Vorrichtung 10 aus Fig. 3 vorgesehen ist.

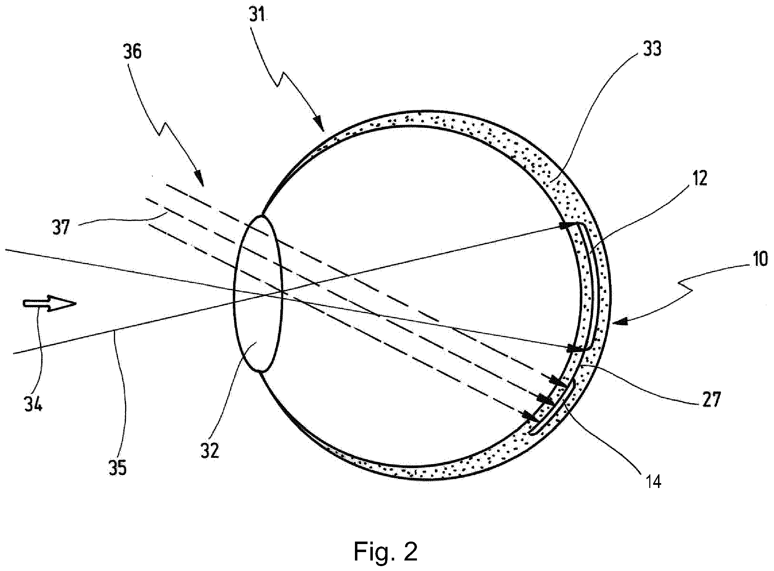

Die Vorrichtung 10 aus Fig. 1 ist dazu bestimmt, vollständig in ein menschliches Auge 31 implantiert zu werden, das in Fig. 2 sehr schematisch dargestellt ist. Der Einfachheit halber sind nur die Linse 32 sowie die Retina 33 gezeigt, in die die Vorrichtung 10 eingepflanzt wurde. Die Vorrichtung 10 wird dabei vorzugsweise in den so genannten subretinalen Raum eingebracht, der sich zwischen dem Pigment-Epithel und der Fotorezeptorschicht bildet. Sofern die Fotorezeptorschicht degeneriert oder verloren ist, bildet sich der subretinale Raum zwischen dem Pigment-Epithel und der Schicht der Bipolar- und Horizontalzellen. Die Vorrichtung 10 wird dabei so platziert, dass über die in Fig. 1 gezeigten Stimulationselektroden 22 Stimulationssignale auf Zellen in der Retina 33 ausgeübt werden können.

Durch einen Pfeil angedeutetes sichtbares Licht 34, dessen Strahlengang bei 35 zu sehen ist, wird über die Linse 32 auf die Stimulationseinheit 12 geleitet, wo das sichtbare Licht 34 in elektrische Signale umgewandelt wird, die über die Verstärker 21 aus Fig. 1 in Stimulationssignale gewandelt werden.

In Fig. 2 ist zu erkennen, dass die Versorgungseinheit 14 außerhalb des Einfallbereiches des sichtbaren Lichtes 34 liegt. Auf die Versorgungseinheit 14 ist Fremdenergie 36 in Form von Strahlen von IR-Licht 37 gerichtet, das in dem IR-Empfänger 15 in eine elektrische Spannung umgewandelt wird, die über die Mehrfachleitung 26 zu der Spannungserzeugung 17 gelangt, wo aus ihr entsprechende Versorgungsspannungen erzeugt werden. Diese Versorgungsspannungen gelangen dann über die Leiterbahnen 27 zu der Stimulationseinheit 12, wo sie verwendet werden, um Bauteile zu versorgen, die das einfallende, sichtbare Licht 34 in Stimulationssignale umzuwandeln.

In Fig. 3 ist eine weitere Vorrichtung 10 zur Elektrostimulation des Auges in nicht maßstabsgetreuer Darstellung gezeigt, bei der die Energieversorgung nicht über eine implantierte Versorgungseinheit 14 erfolgt, die durch eingekoppeltes IR-Licht mit Energie versorgt wird, sondern über eine externen Versorgungseinheit 41, die beispielsweise induktiv mit Energie versorgt wird oder eine Batterie enthält bzw. auf nicht gezeigte Weise mit einer Batterie oder einer Gleichspannungsquelle verbunden ist.

Die externe Versorgungseinheit 41 ist mechanisch und elektrisch mit einem Verbindungsband 42 verbunden, das an seinem anderen Ende mit der schon aus Fig. 1 bekannten Stimulationseinheit 12 verbunden ist. Die externe Versorgungseinheit 41 wird außerhalb des Auges beispielsweise am Schädel des Patienten befestigt. Über die Versorgungseinheit 41 wird elektrische Energie zu der Stimulationseinheit 12 gesandt, wobei gleichzeitig auch Steuersignale übermittelt werden können, die die Funktionsweise der Stimulationseinheit so beeinflussen, wie dies beispielsweise in der WO 2005/000395 A1 beschrieben ist, deren Inhalt hiermit zum Gegenstand der vorliegenden Anmeldung gemacht wird.

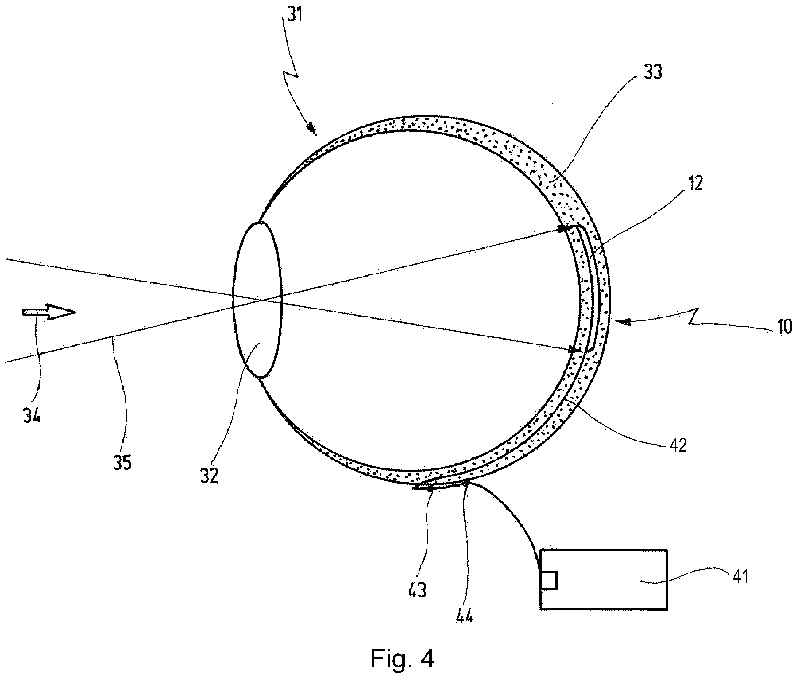

Etwa 50 mm von der Stimulationseinheit 12 entfernt sind an dem Verbindungsband 42 Befestigungslaschen 43 und 44 vorgesehen, über die das Verbindungsband 42 unverrückbar außen auf der Sclera des Auges befestigt wird, wie dies in Fig. 4 schematisch gezeigt ist.

Fig. 4 ist eine Darstellung wie Fig. 2, jedoch jetzt für die Vorrichtung gemäß Fig. 3. Es ist zu erkennen, dass das Verbindungsband 42 seitlich aus dem Auge herausgeführt und dort außen auf der Sclera mit den Befestigungslaschen 43 und 44 befestigt wird, bevor das Verbindungsband 42 weiter zu der externen Versorgungseinheit 41 führt. Auf diese Weise ist dafür gesorgt, dass bei Bewegungen des Auges 31 die Stimulationseinheit 12 unverrückbar in der Retina 33 gehalten wird.

Es sei noch erwähnt, dass die Abmaße insbesondere der Stimulationseinheit 12, der Befestigungslaschen 43, 44, des Verbindungsbandes 42 sowie der externen Versorgungseinheit 41 in den Fig. 3 und 4 weder maßstäblich noch in richtiger Größenrelation zueinander dargestellt sind.

Ein zu dem Verbindungband 42 vergleichbares Verbindungband kann auch bei der Vorrichtung 10 aus Fig. 1 verwendet werden, um Stimulationseinheit 12 und Versorgungseinheit 14 miteinander zu verbinden. Das Verbindungsband 42 kann auch auf der Folie 11 ausgebildet sein.

In jedem Fall befindet sich das Verbindungsband 42 nach der Implantation der Stimulationseinheit 12 zumindest teilweise im Körper des Patienten, wo es mit Körperflüssigkeit in Kontakt gelangt, was im Stand der Technik schon nach kurzer Verweildauer zu den eingangs geschilderten Problemen, also zu einem Kurzschluss oder einer Leiterbahnunterbrechung führt.

Ähnliche Probleme ergeben sich bei anderen Vorrichtungen, die zum Zweck der Kontaktierung und/oder Elektrostimulation von biologischem Gewebe zur zumindest teilweisen Implantation in den menschlichen oder tierischen Körper vorgesehen sind.

Auch solche Vorrichtungen umfassen eine zur Implantation vorgesehene erste Einheit und eine zweite Einheit, die entweder auch implantiert wird oder außerhalb des Körpers verbleibt, wobei die beiden Einheiten auf einem gemeinsamen Substrat oder auf verschiedenen Substraten ausgebildet sind. Zwischen den beiden Einheiten verlaufen auch hier zumindest zwei Leiterbahnen, die in die Spannungsversorgung eingebunden sind und auf unterschiedlichen Spannungspotentialen, beispielsweise auf Masse bzw. Vcc liegen.

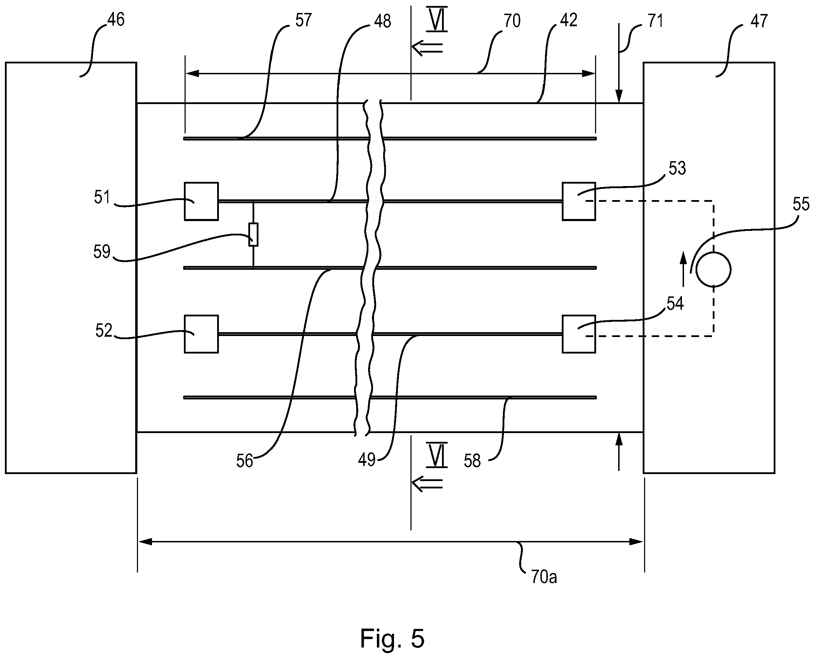

In Fig. 5 ist das Verbindungsband 42 aus Fig. 3 in schematischer Draufsicht gezeigt, wie es eine implantierbare erste Einheit 46 und eine der Energieversorgung der ersten Einheit 46 dienende zweite Einheit 47 miteinander verbindet. Beide Einheiten 46, 47 sind mechanisch und elektrisch mit dem Verbindungsband 42 verbunden.

Auf dem Verbindungsband 42 sind zwei Leiterbahnen 48, 49 angeordnet, die an ihren äußeren Enden mit Anschlussflächen 51, 52, 53 und 54 verbunden sind, von denen nicht gezeigte Verbindungen zu der ersten bzw. zweiten Einheit 46, 47 gehen, so dass zum Zwecke der Spannungsversorgung eine der beiden Leiterbahnen 48, 49 auf Massepotential und die andere auf dem Potential Vcc der Versorgungsgleichspannung liegt, die beispielsweise 3 Volt beträgt. In Fig. 5 ist mit 55 eine in der zweiten Einheit 47 zu diesem Zweck vorgesehene Spannungsversorgung angedeutet, die beispielsweise der Spannungserzeugung 17 aus der Versorgungseinheit 14 entspricht.

Zwischen den beiden parallel zueinander verlaufenden Leiterbahnen 48, 49 verläuft eine zu diesen parallele Zusatzleiterbahn 56, die weder mit der ersten oder mit der zweiten Einheit 48, 49, noch mit einer der beiden Leiterbahnen 48, 49 elektrisch verbunden ist. Die Zusatzleiterbahn 56 ist somit nicht in die Versorgung der ersten Einheit 47 mit Energie eingebunden und folglich insoweit elektrisch funktionslos.

Neben der ersten Leiterbahn 48 verläuft parallel eine zweite Zusatzleiterbahn 57 und neben der zweiten Leiterbahn 49 parallel eine dritte Zusatzleiterbahn 58, so dass die Leiterbahnen 48, 49 jeweils zwischen zwei Zusatzleiterbahnen 56 und 57 bzw. 56 und 58 verlaufen. Auch die zweite und dritte Zusatzleiterbahn 57, 58 sind elektrisch funktionslos.

Für den Fall, dass derart floatende Zusatzleiterbahnen 56, 57, 58 aus elektronischen Gründen nicht akzeptierbar sind, können sie hochohmig mit Masse verbunden werden, wie dies in Fig. 5 durch einen Widerstand 59 angedeutet ist, der elektrisch mit der auf Masse liegenden Leiterbahn 48 und der Zusatzleiterbahn 56 verbunden ist und einen Widerstandswert von 1 Megaohm aufweist.

Die Leiterbahnen 48, 49 sowie die Zusatzleiterbahnen 56, 57, 58 bestehen aus Edelmetall, im vorliegenden Beispiel aus aufgedruckten Leiterbahnen aus Gold.

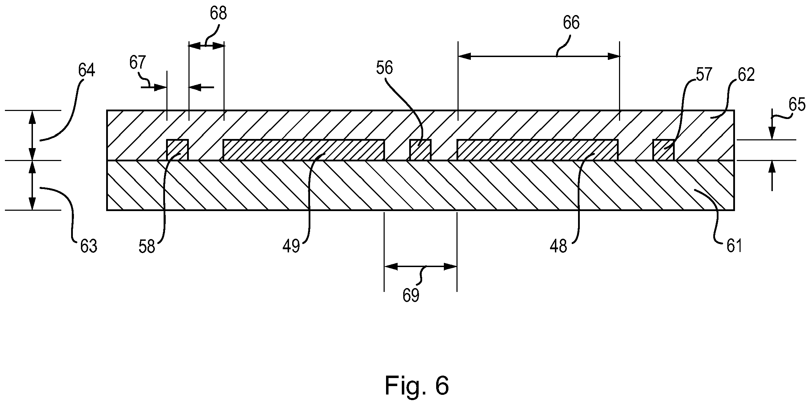

In Fig. 6 ist ein Schnitt durch das Verbindungsband 42 längs der Linie VI - VI aus Fig. 5 gezeigt. Das Verbindungsband 42 weist eine untere, isolierende Materialschicht 61 auf, auf der die Leiterbahnen 48, 49 und die Zusatzleiterbahnen 56, 57, 58 angeordnet sind, die von einer oberen, isolierenden Materialschicht 62 bedeckt sind. Beide Materialschichten 61, 62 bestehen in diesem Beispiel aus Polyimid.

Die obere Materialschicht 62 füllt die Zwischenräume zwischen den Leiterbahnen 48, 48 und den Zusatzleiterbahnen 56, 57, 58 und liegt dort sowie außen neben den Zusatzleiterbahnen 57 und 58 auf der unteren Materialschicht 61 auf und ist mechanisch mit ihr verbunden.

Die Materialschichten 61, 62 weisen jeweils eine Dicke 63, 64 auf, die bei ca. 7 um (Mikrometer) liegt. Die Leiterbahnen 48, 49 und Zusatzleiterbahnen 56, 57, 58 weisen jeweils eine Dicke 65 auf, die bei ca. 3,5 um liegt. Die Leiterbahnen 48, 49 weisen eine Breite 66 von jeweils 100 um und die Zusatzleiterbahnen 56, 57, 58 jeweils eine Breite 67 von 20 um auf, wobei zwischen den Leiterbahnen 48, 49 und Zusatzleiterbahnen 56, 57, 58 ein Abstand 68 bzw. 69 von jeweils 20 um vorhanden ist.

Aus Fig. 5 ist zu entnehmen, dass die Zusatzleiterbahnen 56, 57, 58 eine bei 70 angedeutete Länge von 50 mm aufweisen, die zumindest der Länge 70a der Leiterbahnen 48 und 49 zwischen den Anschlussflächen 51 und 53 bzw. 52 und 54 entspricht.

Das Verbindungsband 42 weist in dem Beispiel der Fig. 5 eine Länge 70a von ca. 70 mm und eine Breite 71 von 5 mm auf.

Ohne die Zusatzleiterbahnen 56, 57, 58 würden die Leiterbahnen 48 und 49 einige 100 - jedoch nicht mehr als etwa 1000 - Stunden nach der Implantation in einen menschlichen oder tierischen Körper einen Kurzschluss bilden oder eine Leiterbahnunterbrechung zeigen, so dass die Versorgung der ersten Einheit 46 mit elektrischer Energie unterbrochen wird.

Auf bisher nicht abschließend aufgeklärte Weise sorgt schon eine hochohmige bzw. an ihren beiden Enden nicht angeschlossene Zusatzleiterbahn 56 zwischen den beiden Leiterbahnen 48 und 49 dafür, dass die Verweildauer des Verbindungsbandes 42 in physiologischer Umgebung deutlich verlängert werden kann. Dieser Effekt beruht nicht vorrangig darauf, dass der Abstand 69 zwischen den beiden Leiterbahnen 48 und 49 durch das Einfügen der Zusatzleiterbahn 56 vergrößert wurde.

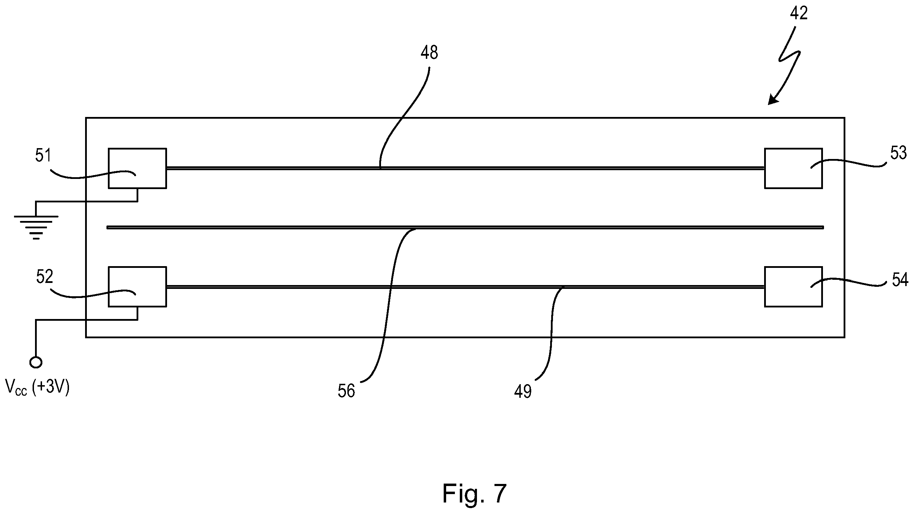

In Fig. 7 ist ein Versuchsaufbau für Langzeitversuche mit einem Verbindungsband 42 gezeigt, bei dem die Leiterbahn 48 an ihrer Anschlussfläche 51 auf Masse und die Leiterbahn 49 an ihrer Anschlussfläche 52 auf Spannungspotential von + 3 Volt gelegt und das Verbindungsband 42 dann vollständig bedeckt in PBS-Puffer (phosphate buffered saline; Dulbecco's PBS ohne Calcium und ohne Magnesium) bei 37° C gelagert wurde.

In einem Langzeitversuch wurde dann an den Anschlussflächen 53 und 54 der Leckstrom zwischen den Leiterbahnen 48, 49 gemessen. In diesem Langzeitversuch wurden Verbindungsbänder 42 mit Leiterbahnen 48, 49 unterschiedlicher Breite 66 und unterschiedlicher Abstände 69 jeweils mit und ohne Zusatzleiterbahn 56 untersucht, um die Zeit bis zum Kurzschluss zwischen den beiden Leiterbahnen 48 und 49 zu bestimmen.

In einer ersten Versuchsreihe wurden keine Zusatzleiterbahnen 56, 57, 58 vorgesehen und Leiterbahnen 48, 49 mit Breiten 66 von 75, 100, 125, 150, 175 und 200 um (Mikrometer) untersucht, wobei der Abstand 69 jeweils der Breite 66 der Leiterbahnen 48, 49 entsprach. Die Zeit bis zu einem Kurzschluss zwischen den Leiterbahnen 48, 49 lag zwischen teilweise weniger als 100 Stunden bis maximal bei nahezu1000 Stunden, variierte aber für unterschiedliche Testobjekte einer Breite 66 so stark, dass keine statistisch signifikante Korrelation zwischen der Breite 66 und der maximalen Verweildauer zu erkennen war.

In einer zweiten Versuchsreihe wurde zwischen den Leiterbahnen 48, 49 eine Zusatzleiterbahn 56 angeordnet, die eine Breite 67 von 50 um und einen Abstand 68 zu den benachbarten Leiterbahnen 48, 49 von 75 um aufwies.

Diese Langzeitversuche wurden für Leiterbahnen 48, 49 mit einer Breite 68 von 100 um und 125 um durchgeführt. Die Verweildauer war signifikant größer als bei den Testobjekten ohne Zusatzleiterbahn, wobei zudem die statistische Streuung deutlich geringer ausfiel. Es wurden Verweilzeiten länger als 2500 Stunden und länger als 4000 Stunden gemessen, bevor einige Versuche bei einer Laufzeit von 4000 Stunden abgebrochen wurden.

Dieser Effekt lässt sich auch bereits bewirken, wenn die Länge 70 der Zusatzleiterbahn 56 zumindest 50% der Länge der Leiterbahnen 48, 49 zwischen den Anschlussflächen 51 und 53 bzw. 52 und 54 beträgt. Wenn die Länge 70 dagegen 90% dieser Länge beträgt, ist der Effekt noch einmal deutlich verbessert.

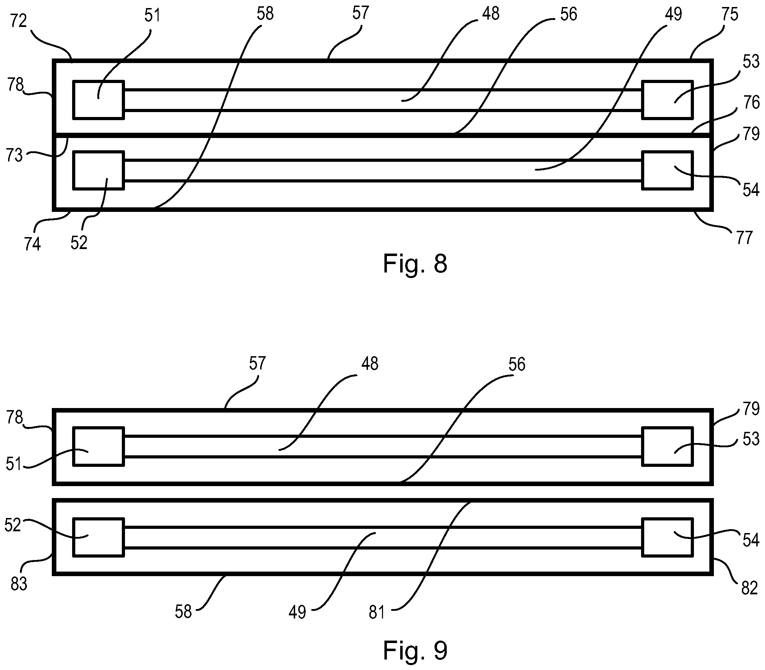

Um die Verweildauer weiter positiv zu beeinflussen, können die Leiterbahnen 48 und 49 mit einer zweiten bzw. dritten Zusatzleiterbahn 57 bzw. 58 umgeben werden, wie es in Fig. 5 gezeigt ist. Die Zusatzleiterbahnen 56, 56, 58 können sich mit ihren Enden 72, 73, 74, 75, 76, 77 über die Anschlussflächen 51, 52, 53 und 54 hinaus erstrecken und dort über Querbahnen 78 und 79 miteinander verbunden sein, wie es in Fig. 8 gezeigt ist.

Zwischen den Leiterbahnen 48 und 49 kann zudem eine weitere parallel zu diesen verlaufende Zusatzleiterbahn 81 parallel zu der Zusatzleiterbahn 56 angeordnet sein, wobei auch in dieser Konstruktion die Zusatzleiterbahnen 56 und 57 über Querbahnen 78 und 79 sowie die Zusatzleiterbahnen 81 und 58 durch Querbahnen 82 und 83 miteinander verbunden sind, wie es in Fig. 9 gezeigt ist.

Die Querbahnen 78, 79, 81 weisen dieselbe Breite 67 und Dicke 65 auf wie die Zusatzleiterbahnen 56, 57, 58, 81.

Weil die Materialschichten 61, 62 optisch transparent sind, sind die Leiterbahnen 48, 49 sowie die Zusatzleiterbahnen 56, 57, 58, 81 in der Draufsicht der Fig. 7 bis 9 zu erkennen.

## Patentansprüche

1. Vorrichtung zur Kontaktierung und/oder Elektrostimulation von biologischem Gewebe, mittels zumindest einer Elektrode, wobei die Vorrichtung (10) zumindest eine erste Einheit (12; 46), an der die zumindest eine Elektrode vorgesehen ist, und die zur Implantation in einen menschlichen oder tierischen Körper ausgelegt ist, eine zweite Einheit (14; 41; 47), die eine Spannungsversorgung (55) zur Versorgung der ersten Einheit (12; 46) mit elektrischer Energie bereitstellt, sowie zumindest eine erste und eine zweite Leiterbahn (48, 49) zur Verbindung der Spannungsversorgung (55) mit der ersten Einheit (12; 46) aufweist, wobei die Leiterbahnen (48, 49) jeweils elektrisch mit der ersten (12, 46) und zweiten Einheit (14; 41; 47) verbunden sind und auf unterschiedlichen Spannungspotentialen liegen, wobei die erste Einheit (46) eine Stimulationseinheit (12) für die Retina (33) ist, die eine Vielzahl von Bildelementen (18) umfasst, die einfallendes Licht in elektrische Signale umwandeln, die über Stimulationselektroden (22) abgegeben werden, und **dadurch gekennzeichnet, dass** zumindest eine erste, funktional nicht in die Versorgung der ersten Einheit (12; 46) mit elektrischer Energie eingebundene Zusatzleiterbahn (56) vorgesehen ist, die räumlich zwischen der ersten und der zweiten Leiterbahn (48, 49) angeordnet ist.

2. Vorrichtung nach Anspruch 1, **dadurch gekennzeichnet, dass** die Zusatzleiterbahn (56) potentialfrei geschaltet ist.

3. Vorrichtung nach Anspruch 1, **dadurch gekennzeichnet, dass** die Zusatzleiterbahn (56) gegenüber der ersten (48) oder zweiten (49) Leiterbahn einen elektrischen Widerstand (59) aufweist, der größer als 100 kOhm ist.

4. Vorrichtung nach einem der Ansprüche 1 bis 3, **dadurch gekennzeichnet, dass** die Zusatzleiterbahn (56) über zumindest 50 % der Länge der ersten und zweiten Leiterbahn (48, 49) zwischen diesen verläuft.

5. Vorrichtung nach Anspruch 4, **dadurch gekennzeichnet, dass** die Zusatzleiterbahn (56) über zumindest 90 % der Länge der ersten und zweiten Leiterbahn (48, 49) zwischen diesen verläuft.

6. Vorrichtung nach Anspruch 5, **dadurch gekennzeichnet, dass** die Zusatzleiterbahn (56) über zumindest der gesamten Länge der ersten und zweiten Leiterbahn (48, 49) zwischen diesen verläuft.

7. Vorrichtung nach einem der Ansprüche 1 bis 6, **dadurch gekennzeichnet, dass** neben der ersten Leiterbahn (48) eine zweite Zusatzleiterbahn (57) derart vorgesehen ist, dass die erste Leiterbahn (48) zwischen der ersten und der zweiten Zusatzleiterbahn (56, 57) verläuft.

8. Vorrichtung nach Anspruch 7, **dadurch gekennzeichnet, dass** neben der zweiten Leiterbahn (49) eine dritte Zusatzleiterbahn (58) derart vorgesehen ist, dass die zweite Leiterbahn (49) zwischen der ersten und der dritten Zusatzleiterbahn (56, 58) verläuft.

9. Vorrichtung nach Anspruch 7 oder Anspruch 8, **dadurch gekennzeichnet, dass** zumindest zwei Zusatzleiterbahnen (56, 57; 56, 58) an ihren äußeren Enden (72, 73, 74; 75, 76, 77) miteinander verbunden sind.

10. Vorrichtung nach einem der Ansprüche 1 bis 9, **dadurch gekennzeichnet, dass** zwischen der ersten und der zweiten Leiterbahn (48, 49) eine weitere Zusatzleiterbahn (81) vorgesehen ist.

11. Vorrichtung nach Anspruch 8 und Anspruch 10, **dadurch gekennzeichnet, dass** die erste und die zweite Zusatzleiterbahn (56, 57) sowie die weitere und die dritte Zusatzleiterbahn (81, 58) an ihren äußeren Enden miteinander verbunden sind.

12. Vorrichtung nach einem der Ansprüche 1 bis 11, **dadurch gekennzeichnet, dass** die oder jede Zusatzleiterbahn (56, 57, 58, 81) eine Breite (67) aufweist, die geringer ist als die Breite (66) der Leiterbahnen (48, 49), vorzugsweise weniger als 20% der Breite (66) der Leiterbahnen (48, 49) beträgt.

13. Vorrichtung nach einem der Ansprüche 1 bis 12, **dadurch gekennzeichnet, dass** die Leiterbahnen (48, 49) und die Zusatzleiterbahn (56) oder die Zusatzleiterbahnen (56, 57, 58, 81) eine Dicke (65) aufweisen, die zwischen 0,1 um und 20 um liegt, und dass die Leiterbahnen (48, 49) eine Breite aufweisen, die zwischen 10 um und 1 mm liegt.

14. Vorrichtung nach einem der Ansprüche 1 bis 13, **dadurch gekennzeichnet, dass** sie ein flexibles Verbindungsband (42) umfasst, das mechanisch mit der ersten (12; 46) und der zweiten Einheit 14; 41; 47) verbunden ist und auf dem die Leiterbahnen (48, 49) sowie die Zusatzleiterbahn (56) oder die Zusatzleiterbahnen (56, 57, 58, 81) angeordnet sind.

15. Vorrichtung nach einem der Ansprüche 1 bis 14, **dadurch gekennzeichnet, dass** die Leiterbahnen (48, 49) und die Zusatzleiterbahn (56) oder die Zusatzleiterbahnen (56, 57, 58, 81) parallel zueinander verlaufen.

16. Vorrichtung nach einem der Ansprüche 1 bis 15, **dadurch gekennzeichnet, dass** die erste Leiterbahn (48) mit einer Masse und die zweite Leiterbahn (49) mit einer Gleichspannung einer Versorgungsspannung verbunden ist.

17. Vorrichtung nach einem der Ansprüche 14 bis 16, **dadurch gekennzeichnet, dass** sie eine untere, elektrisch isolierende Materialschicht (61), auf der die Leiterbahnen (48, 49) und die Zusatzleiterbahn (56) oder die Zusatzleiterbahnen (56, 57, 58, 81) untergebracht sind, und eine über den Leiterbahnen (48, 49) und der Zusatzleiterbahn (56) oder den Zusatzleiterbahnen (56, 57, 58, 81) vorgesehene obere, elektrisch isolierende Materialschicht (62) aufweist.

18. Vorrichtung nach Anspruch 17, **dadurch gekennzeichnet, dass** die isolierenden Materialschichten (61, 62) aus Polyimid gefertigt sind.

19. Vorrichtung nach einem der Ansprüche 1 bis 18, **dadurch gekennzeichnet, dass** die zweite Einheit (47) eine implantierbare Versorgungseinheit (14) ist, die einfallende Strahlungsenergie in elektrische Energie zur Versorgung der Stimulationseinheit (12) umwandelt.

20. Vorrichtung nach einem der Ansprüche 1 bis 18, **dadurch gekennzeichnet, dass** die zweite Einheit (47) eine externe Versorgungseinheit (41) ist, die elektrische Energie zur Versorgung der Stimulationseinheit (12) bereitstellt.

## Claims

1. Device for contacting and/or electrostimulation of biological tissue, by means of at least one electrode, wherein the device (10) has at least a first unit (12; 46), on which the at least one electrode is provided and which is configured for implantation in a human or animal body, a second unit (14; 41; 47), which provides a voltage supply (55) for supplying the first unit (12; 46) with electrical energy, and at least a first and a second conductive track (48, 49) for connecting the voltage supply (55) to the first unit (12; 46), the conductive tracks (48, 49) respectively being electrically connected to the first unit (12; 46) and second unit (14; 41; 47) and being at different voltage potentials, wherein the first unit (46) comprises a stimulation unit (12) for the retina (33), which unit comprises a multiplicity of pixel elements (18) that convert incident light into electrical signals that are emitted via stimulation electrodes (22),
**characterized in that** at least a first additional conductive track (56) functionally not involved in the supply of the first unit (12; 46) with electrical energy is provided, which first additional conductive track is arranged spatially between the first and second conductive tracks (48, 49).

2. Device according to Claim 1, **characterized in that** the additional conductive track (56) is connected potential-free.

3. Device according to Claim 1, **characterized in that** the additional conductive track (56) has an electrical resistance (59) relative to the first (48) or second (49) conductive tracks which is more than 100 kOhm.

4. Device according to anyone of Claims 1 to 3, **characterized in that** the additional conductive track (56) extends between the first and second conductive tracks (48, 49) over at least 50% of their length.

5. Device according to Claim 4, **characterized in that** the additional conductive track (56) extends between the first and second conductive tracks (48, 49) over at least 90% of their length.

6. Device according to Claim 5, **characterized in that** the additional conductive track (56) extends between the first and second conductive tracks (48, 49) over at least their entire length.

7. Device according to anyone of Claims 1 to 6, **characterized in that** a second additional conductive track (57) is provided beside the first conductive track (48), in such a way that the first conductive track (48) extends between the first and second additional conductive tracks (56, 57).

8. Device according to Claim 7, **characterized in that** a third additional conductive track (58) is provided beside the second conductive track (49), in such a way that the second conductive track (49) extends between the first and third additional conductive tracks (56, 58).

9. Device according to Claim 7 or Claim 8, **characterized in that** at least two additional conductive tracks (56, 57; 56, 58) are connected to one another at their outer ends (72, 73, 74; 75, 76, 77).

10. Device according to one of Claims 1 to 9, **characterized in that** a further additional conductive track (81) is provided between the first and second conductive tracks (48, 49).

11. Device according to Claim 8 and Claim 10, **characterized in that** the first and second additional conductive tracks (56, 57) and the further and the third additional conductive tracks (81, 58) are connected to one another at their outer ends.

12. Device according to anyone of Claims 1 to 11, **characterized in that** the or each additional conductive track (56, 57, 58, 81) has a width (67) which is less than the width (66) of the conductive tracks (48, 49), preferably less than 20% of the width (66) of the conductive tracks (48, 49).

13. Device according to anyone of Claims 1 to 12, **characterized in that** the conductive tracks (48, 49) and the additional conductive track (56) or the additional conductive tracks (56, 57, 58, 81) have a thickness (65) which lies between 0.1 µm and 20 µm, and **in that** the conductive tracks (48, 49) have a width which lies between 10 µm and 1 mm.

14. Device according to anyone of Claims 1 to 13, **characterized in that** it comprises a flexible connecting band (42) which is mechanically connected to the first unit (12; 46) and the second unit (14; 41; 47) and on which the conductive tracks (48, 49) as well as the additional conductive track (56) or the additional conductive tracks (56, 57, 58, 81) are arranged.

15. Device according to anyone of Claims 1 to 14, **characterized in that** the conductive tracks (48, 49) and the additional conductive track (56) or the additional conductive tracks (56, 57, 58, 81) extend parallel to one another.

16. Device according to anyone of Claims 1 to 15, **characterized in that** the first conductive track (48) is connected to ground and the second conductive track (49) is connected to a DC voltage of a supply voltage.

17. Device according to anyone of Claims 14 to 16, **characterized in that in that** it has a lower electrically insulating material layer (61), on which the conductive tracks (48, 49) and the additional conductive track (56) or the additional conductive tracks (56, 57, 58, 81) are fitted, and an upper electrically insulating material layer (62) provided above the conductive tracks (48, 49) and the additional conductive track (56) or the additional conductive tracks (56, 57, 58, 81).

18. Device according to Claim 17, **characterized in that** the insulating material layers (61, 62) are made of polyimide.

19. Device according to anyone of Claims 1 to 18, **characterized in that** the second unit (47) is an implantable supply unit (14) which converts incident radiation energy into electrical energy for supplying the stimulation unit (12).

20. Device according to anyone of Claims 1 to 18, **characterized in that** the second unit (47) is an external supply unit (41) which provides electrical energy for supplying the stimulation unit (12).

## Revendications

1. Dispositif destiné à la mise en contact électrique et/ou l'électrostimulation de tissus biologiques, au moyen d'au moins une électrode, dans lequel le dispositif (10) présente au moins une première unité (12 ; 46) sur laquelle l'au moins une électrode est prévue, et qui est conçu pour l'implantation dans un corps humain ou animal, une deuxième unité (14 ; 41 ; 47) qui procure une alimentation électrique (55) pour alimenter la première unité (12 ; 46) avec une énergie électrique, et au moins une première et une deuxième piste conductrice (48, 49) pour relier l'alimentation électrique (55) à la première unité (12 ; 46), dans lequel les pistes conductrices (48, 49) sont respectivement reliées électriquement à la première (12, 46) et la deuxième unité (14 ; 41 ; 47) et sont sur des potentiels de tension différents, dans lequel la première unité (46) est une unité de stimulation (12) pour la rétine (33) qui comprend une multiplicité d'éléments d'image (18) qui convertissent une lumière incidente en signaux électriques qui sont délivrés par des électrodes de stimulation (22), et **caractérisé en ce qu'**au moins une première piste conductrice supplémentaire (56), fonctionnellement non liée à l'approvisionnement de la première unité (12 ; 46) avec de l'énergie électrique, est prévue, qui est disposée spatialement entre la première et la deuxième piste conductrice (48, 49).

2. Dispositif selon la revendication 1, **caractérisé en ce que** la piste conductrice supplémentaire (56) est montée sans potentiel.

3. Dispositif selon la revendication 1, **caractérisé en ce que** la piste conductrice supplémentaire (56) présente par rapport à la première piste conductrice (48) ou la deuxième piste conductrice (49) une résistance électrique (59) qui est supérieure à 100 kiloohms.

4. Dispositif selon l'une des revendications 1 à 3, **caractérisé en ce que** la piste conductrice supplémentaire (56) évolue sur au moins 50 % de la longueur de la première et de la deuxième piste conductrice (48, 49) entre celles-ci.

5. Dispositif selon la revendication 4, **caractérisé en ce que** la piste conductrice supplémentaire (56) évolue sur au moins 90 % de la longueur de la première et de la deuxième piste conductrice (48, 49) entre celles-ci.

6. Dispositif selon la revendication 5, **caractérisé en ce que** la piste conductrice supplémentaire (56) évolue sur au moins la longueur totale de la première et de la deuxième piste conductrice (48, 49) entre celles-ci.

7. Dispositif selon l'une des revendications 1 à 6, **caractérisé en ce que**, parallèlement à la première piste conductrice (48) est prévue une deuxième piste conductrice supplémentaire (57) de telle sorte que la première piste conductrice (48) évolue entre la première et la deuxième piste conductrice supplémentaire (56, 58).

8. Dispositif selon la revendication 7, **caractérisé en ce que**, parallèlement à la deuxième piste conductrice (49) est prévue une troisième piste conductrice supplémentaire (58) de telle sorte que la deuxième piste conductrice (49) évolue entre la première et la troisième piste conductrice supplémentaire (56, 57).

9. Dispositif selon la revendication 7 ou la revendication 8, **caractérisé en ce qu'**au moins deux pistes conductrices supplémentaires (56, 57 ; 56, 58) sont reliées les unes aux autres à leurs extrémités extérieures (72, 73, 74 ; 75, 76, 77).

10. Dispositif selon l'une des revendications 1 à 9, **caractérisé en ce qu'**entre la première et la deuxième piste conductrice (48, 49), une autre piste conductrice supplémentaire (81) est prévue.

11. Dispositif selon la revendication 8 et la revendication 10, **caractérisé en ce que** la première et la deuxième piste conductrice supplémentaire (56, 57) ainsi que l'autre et la troisième piste conductrice supplémentaire (81, 58) sont reliées les unes aux autres à leurs extrémités extérieures.

12. Dispositif selon l'une des revendications 1 à 11, **caractérisé en ce que** la ou chaque piste conductrice supplémentaire (56, 57, 58, 81) présente une largeur (67) qui est plus réduite que la largeur (66) des pistes conductrices (48, 49), de préférence inférieure à 20 % de la largeur (66) des pistes conductrices (48, 49).

13. Dispositif selon l'une des revendications 1 à 12, **caractérisé en ce que** les pistes conductrices (48, 49) et la piste conductrice supplémentaire (56) ou les pistes conductrices supplémentaires (56, 57, 58, 81) présentent une épaisseur (65) qui se situe entre 0,1 µm et 20 µm, et **en ce que** les pistes conductrices (48, 49) présentent une largeur qui se situe entre 10 µm et 1 mm.

14. Dispositif selon l'une des revendications 1 à 13, **caractérisé en ce qu'**il comprend une bande de liaison flexible (42) qui est reliée mécaniquement avec la première (12 ; 46) et la deuxième unité (14 ; 41 ; 47) et sur laquelle sont disposées les pistes conductrices (48, 49) ainsi que la piste conductrice supplémentaire (56) ou les pistes conductrices supplémentaires (56, 57, 58, 81).

15. Dispositif selon l'une des revendications 1 à 14, **caractérisé en ce que** les pistes conductrices (48, 49) et la piste conductrice supplémentaire (56) ou les pistes conductrices supplémentaires (56, 57, 58, 81) évoluent parallèlement les unes aux autres.

16. Dispositif selon l'une des revendications 1 à 15, **caractérisé en ce que** la première piste conductrice (48) est reliée à une masse et la deuxième piste conductrice (49) est reliée à une tension continue d'une tension d'alimentation.

17. Dispositif selon l'une des revendications 14 à 16, **caractérisé en ce qu'**il présente une couche de matériau inférieure, électriquement isolante (61) sur laquelle les pistes conductrices (48, 49) et la piste conductrice supplémentaire (56) ou les pistes conductrices supplémentaires (56, 57, 58, 81) sont placées et présente une couche de matériau prévue supérieure, électriquement isolante (62) de la piste conductrice supplémentaire (56) ou des pistes conductrices supplémentaires (56, 57, 58, 81).

18. Dispositif selon la revendication 17, **caractérisé en ce que** les couches de matériau isolantes (61, 62) sont en polyimide.

19. Dispositif selon l'une des revendications 1 à 18, **caractérisé en ce que** la deuxième unité (47) est une unité d'alimentation implantable (14) qui convertit l'énergie de rayonnement incidente en énergie électrique pour l'alimentation de l'unité de stimulation (12).

20. Dispositif selon l'une des revendications 1 à 18, **caractérisé en ce que** la deuxième unité (47) est une unité d'alimentation externe (41) qui procure une énergie électrique pour l'alimentation de l'unité de stimulation (12).
